(19) Europäisches Patentamt
European Patent Office
Office européen des brevets

(11) Numéro de publication: **0 049 203**
**A1**

(12) **DEMANDE DE BREVET EUROPEEN**

(21) Numéro de dépôt: **81401497.3**

(22) Date de dépôt: **28.09.81**

(51) Int. Cl.³: **C 07 D 263/58**, A 61 K 31/42

(30) Priorité: **29.09.80 FR 8020861**

(43) Date de publication de la demande: **07.04.82**
**Bulletin 82/14**

(84) Etats contractants désignés: **AT BE CH DE FR GB IT LI LU NL SE**

(71) Demandeur: **Centre Européen de Recherches Mauvernay (CERM), Route de Marsat, F-63203 Riom Cedex (FR)**

(72) Inventeur: **Lesieur, Daniel, 20, rue de Verdun, F-59147 Gondecourt (FR)**
Inventeur: **Lespagnol, Charles, 115, Avenue Pasteur, F-59130 Lambersart (FR)**
Inventeur: **Vaccher, Marie-Pierre, Résidence Sylvère Verhulst 6/51, rue des Catiches, F-59000 Lille (FR)**
Inventeur: **Bonte, Jean-Paul, 20, boulevard Descat, F-59200 Tourcoing (FR)**
Inventeur: **Debaert, Michel, 1, rue Lavoisier, F-59000 Lille (FR)**
Inventeur: **Busch, Norbert, "Le Bouquet" Loubeyrat, F-63410 Manzat (FR)**
Inventeur: **Combourieu, Michel, 6, rue du Mont-Mouchet, F-15000 Aurillac (FR)**

(74) Mandataire: **Therond, Gérard Raymond, C.E.R.M Route de Marsat, F-63203 Riom Cedex (FR)**

(54) **Nouvelles benzoxazolinones substituées en 6 par une chaîne aminoalcool ou aminocétone, leur préparation et leur application en thérapeutique.**

(57) Composés de formule:

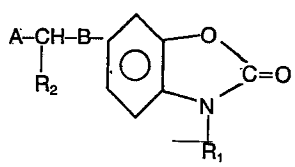

dans laquelle $R_1$ et $R_2$ représentent l'hydrogène ou un radical alkyle inférieur, A représente le radical amino ou un radical aminé secondaire ou tertiaire, ou un radical dans lequel l'atome d'azote de liaison est inclus dans un hétérocyle, et B représente soit le radical carbonyle, soit le radical hydroxyméthylène.

Application au traitement de l'hypertension ou des syndromes douloureux.

EP 0 049 203 A1

# NOUVELLES BENZOXAZOLINONES SUBSTITUEES EN 6 PAR UNE CHAINE AMINOALCOOL OU AMINOCETONE, LEUR PREPARATION ET LEUR APPLICATION EN THERAPEUTIQUE

La présente invention concerne de nouveaux dérivés de la benzoxazolinone substitués en 6 par une chaîne aminoalcool ou aminocétone, ainsi que leurs sels pharmaceutiquement acceptables. L'invention concerne plus particulièrement des dérivés de la benzoxazolinone répondant à la formule générale suivante :

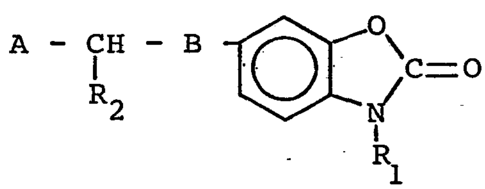

(I)

et leurs sels pharmaceutiquement acceptables, dans laquelle $R_1$ représente l'hydrogène ou un radical alkyle inférieur ; $R_2$ représente l'hydrogène ou un radical alkyle inférieur ; A représente le radical amino ou un radical aminé secondaire ou tertiaire, ou un radical dans lequel l'atome d'azote de liaison est inclus dans un hétérocycle à 5 ou 6 atomes ; B représente soit le radical carbonyle $(-\underset{\underset{O}{\|}}{C}-)$, soit le radical hydroxyméthylène $(-\underset{\underset{OH}{|}}{CH}-)$.

L'invention concerne également l'application des composés de formule I et leurs sels en thérapeutique humaine sous forme de préparation pharmaceutique, notamment en raison de leurs intéressantes propriétés antihypertensives.

Le radical alkyle inférieur, tel que donné dans la définition de $R_1$ et $R_2$ représente de préférence un groupe alkyle ayant 1 à 6 atomes de carbone et plus spécialement un groupe alkyle ayant 1 à 4 atomes de carbone tels que les radicaux méthyl, éthyl, propyl, isopropyl, butyl, terbutyl ou isobutyl.

Par radical aminé secondaire ou tertiaire tel qu'utilisé dans la définition de A, on entend un radical amino dans lequel un ou les deux atomes d'hydrogène ont été remplacés par un radical alkyl inférieur de 1 à 6 atomes de carbone tel que précédemment défini ou par un radical phénylalkyl dans lequel le radical phényl peut être substitué par un ou plusieurs halogènes, un radical alcoxy inférieur ou le radical trifluorométhyl.

Les radicaux aminés secondaires sont par exemple les radicaux méthylamino, éthylamino, propylamino, isopropylamino, n-butylamino, isobutylamino, terbutylamino, benzylamino, phényléthylamino, 3,4-diméthoxy phényléthylamino, 4-fluoro ou 4-chloro phényléthylamino.

Les radicaux aminés tertiaires sont par exemple les radicaux diméthylamino, diéthylamino, dipropylamino, méthyléthylamino et méthylbenzylamino.

Par radical dans lequel l'azote de liaison est inclu dans un hétérocycle à 5 ou 6 atomes, on entend un cycle à 5 ou 6 atomes contenant l'azote, pouvant contenir un autre atome d'oxygène ou d'azote et pouvant éventuellement être substitué par un radical alkyle ayant 1 à 4 atomes, un radical phényl ou benzyl dans lequel le groupe aromatique peut être substitué par un halogène, un radical alcoxy ayant 1 à 4 atomes de carbone, ou le radical trifluorométhyl.

De tels radicaux sont par exemple les radicaux piperidino, morpholino, piperazino, 4-benzylpiperidyl, 4-methylpiperazinyl, 4-phenylpiperazinyl dans lequel le reste phényl peut être substitué par exemple par un radical méthoxy, un atome ou le radical $CF_3$, ou le radical 4-(2-alcoxy-2-phényl-éthyl) pipérazinyl.

Les composés de formule I, et leurs sels pharmaceutiquement acceptables sont préparés selon des procédés connus ou décrits dans la littérature.

Les composés de formule I peuvent par exemple être préparés en condensant une amine A-H et une 6-bromacétyl benzoxazolinone convenablement substituée par $R_1$ et $R_2$, et le cas échéant effectuer la réduction de la cétone obtenue par les moyens habituels pour obtenir les alcools correspondants. Cette synthèse correspond au schéma réactionnel suivant :

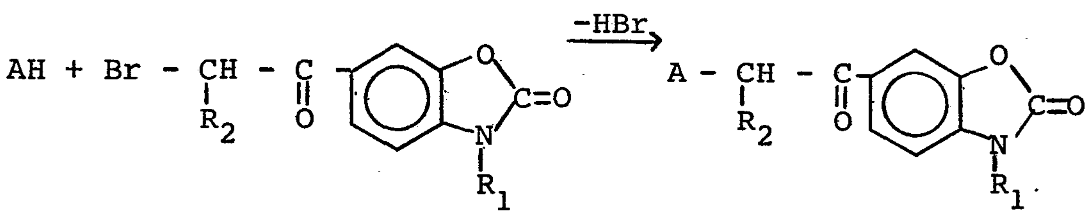


La réaction se déroule au sein d'un solvant organique approprié. On utilise de préférence l'acétone, mais on peut, selon le cas, utiliser d'autres solvants organiques usuels, tels que l'acétonitrile, le benzène, le dioxanne, le chloroforme, le tétrahydrofuranne ou un alcool.

Les deux produits de départ sont mis à réagir dans des proportions telles qu'on ait au moins deux moles d'amine pour une mole de dérivé bromé ; on peut aussi effectuer la réaction en utilisant des quantités équimoléculaires et en ajoutant au milieu réactionnel

un autre composé basique, tel que la triéthylamine ou le carbonate de potassium.

Selon une variante du procédé, on peut obtenir les composés pour lesquels A représente un radical aminé secondaire en préparant d'abord selon la méthode générale sus-indiquée les homologues benzylés (A = N (alkyl)-$CH_2$-$C_6H_5$), puis en traitant ces composés par l'hydrogène en présence de catalyseur (Pd/charbon). Les amines primaires ou secondaires de formule I ainsi obtenues peuvent être, si on le souhaite N-alkylées ou N-aralkylées selon les méthodes usuelles.

Dans le cas où A représente le radical amino, les composés correspondants sont avantageusement obtenus en faisant agir sur le dérivé bromacétylé l'hexaméthylène tétramine en présence d'un solvant, en chauffant à reflux et en hydrolisant ensuite par chauffage en milieu acide.

Les composés de formule I, obtenus selon la synthèse précédente ou selon l'une de ses variantes, peuvent en outre si nécessaire être réduits pour obtenir les dérivés hydroxylés, c'est-à-dire les dérivés de formule I dans lesquels B représente $-\underset{OH}{\underset{|}{CH}}-$.

Cette réduction s'effectue par exemple au moyen du borohydrure de sodium qu'on fait agir sur le dérivé carbonylé préalablement dissous dans le méthanol.

Cette réduction peut également s'effectuer par hydrogénation catalytique, sous pression d'hydrogène à une température voisine de 100°C en présence de charbon palladié à 10 %.

Lorsque $R_2$ est différent de l'hydrogène, le carbone portant le substituant $R_2$ est asymétrique et les alcools correspondants ont une conformation soit thréo, soit érythro. Les isomères thréo sont les principaux produits obtenus au moyen d'une réduction par le borohydrure et les isomères érythro sont les principaux produits obtenus par hydrogénation catalytique.
Ces deux configurations des composés de formule I font partie de l'invention.

Les sels pharmaceutiquement acceptables des composés de formule I sont obtenus en traitant ces dérivés aminés par des acides pharmaceutiquement acceptables tels que l'acide chlorhydrique, l'acide maléique, l'acide fumarique, l'acide citrique, etc...

L'activité pharmacologique des composés de l'invention a été révélée par recherche d'une interférence avec les récepteurs adrénergiques $\beta_1$, $\beta_2$ et $\alpha$ in vitro, conformément aux protocoles expérimentaux ci-après.

Récepteurs adrénergiques $\beta_1$ : on recherche l'antagonisme de l'effet inotrope positif de l'isoprénaline sur les récepteurs $\beta_1$ de l'oreillette gauche de cobaye stimulée électriquement.

Récepteurs adrénergiques $\beta_2$ : on recherche l'antagonisme des effets bronchodilatateurs de l'isoprénaline sur les récepteurs $\beta_2$ du muscle trachéal de cobaye.

Récepteurs adrénergiques $\alpha$ : on étudie l'interférence des composés de l'invention sur la contraction du vas déferens isolé du rat, provoquée par des doses cumulatives de noradrénaline.

Les résultats sont exprimés dans le tableau I par les paramètres usuels de pharmacologie moléculaire, selon les critères utilisés par Van Rossum [Arch. Int. Pharmacodyn., 143, 299-330, 1963].

Les essais pharmacologiques ont en outre montré que les composés étudiés ne possédaient pas une toxicité élevée. La DL 50 par voie orale chez la souris se situe entre 800 $mg.kg^{-1}$ et 1200 $mg.kg^{-1}$.

TABLEAU I

| COMPOSE N° | INTERFERENCE AVEC LES RECEPTEURS ADRENERGIQUES | | |
|---|---|---|---|
| | $\beta_1$ | $\beta_2$ | $\alpha$ |
| 9 | $pA_2 = 5,4 \pm 0,39$<br>$pD'_2 = 3,8$ | Agoniste partiel | Inactif |
| 10 | $pA_2 = 5,9 \pm 0,08$<br>$pD'_2 = 4,59 \pm 0,61$ | Agoniste partiel | $pA_2 = 5,3 \pm 0,12$ |
| 13 | $pA_2 = 5,3$ | Inactif | * + 230 % à $10^{-4}$ M/l |
| 16 | $pA_2 = 5,2$ | Inactif | $pA_2 = 4,73 \pm 0,52$ |
| 18 | $pA_2 = 7,46 \pm 0,17$ | $pA_2 = 6,11 \pm 0,10$ | $pA_2 = 6,03 \pm 0,43$ |
| 19 | $pA_2 = 6,65 \pm 0,11$ | $pA_2 = 5,83 \pm 0,40$ | Inactif |
| 22 | $pA_2 = 5,55 \pm 0,50$ | Agoniste partiel | $pA_2 = 5,7 \pm 0,25$ |
| 25 | $pA_2 = 5,7$<br>$pD'_2 = 4,2$ | Inactif | * + 310 % à $10^{-4}$ M/l |

TABLEAU I (Suite)

| COMPOSE N° | INTERFERENCE AVEC LES RECEPTEURS ADRENERGIQUES | | |
|---|---|---|---|
| | $\beta_1$ | $\beta_2$ | $\alpha$ |
| 27 | $pA_2 = 5,65 \pm 0,40$ | $pA_2 = 4,68 \pm 0,16$ | $pA_2 = 6,14 \pm 0,31$ $pD'_2 = 4,73 \pm 0,38$ |
| 28 | $pA_2 = 5,21 \pm 0,43$ $pD'_2 = 4$ | Inactif | Inactif |
| 29 | $pA_2 = 5,53 \pm 0,28$ $pD'_2 = 4,50 \pm 0,21$ | Agoniste partiel | * + 180 % à $10^{-4}$ M/l |
| 30 | $pA_2 = 5,4 \pm 0,50$ | Inactif | * + 140 % à $10^{-4}$ M/l |

* Potentialisation des effets de la noradrénaline

Les résultats rassemblés dans ce tableau montrent que les composés étudiés possèdent dans leur ensemble une intéressante activité $\beta_1$ bloquante, et qu'ils s'individualisent par l'activité qu'ils exercent sur les récepteurs $\beta_2$ et $\alpha$ , traduisant d'une part leur plus ou moins grande cardiosélectivité et leur influence au niveau bronchique, d'autre part leur tropisme vasculaire.

Le composé n° 18 est celui qui se révèle le plus intéressant en raison de son activité $\beta_1$ bloquante élevée, avec une cardiosélectivité de faible intensité et une activité $\alpha$ bloquante.

Les composés n° 27, 22 et 10 présentent sensiblement le même spectre d'activité que le composé n° 18, bien qu'ayant une activité $\beta_1$ bloquante un peu inférieure,

accompagnée d'une action non spécifique pour le composé n° 10.

Ces mêmes activités se retrouvent pour le composé n° 16, mais sans action bronchodilatatrice.

De nombreux autres composés (N° 9, 13, 25, 28 et 30) possèdent une cardiosélectivité importante, voire totale, le composé n° 9 étant en outre bronchodilatateur. Le composé n° 31, qui n'a pas une activité de même niveau que les autres composés de l'invention sur les récepteurs adrénergiques, possède par contre de remarquables propriétés analgésiques révélées par les tests de stimulus thermique et de stimulus chimique.

Dans le test de stimulus thermique [selon EDDY N.B., LEIMBACH D.J. Pharmacol. Exp. Ther., 107, 385-393, (1953)] la DE 50 de ce composé — qui représente la dose de produit qui augmente de 50 % le temps de réaction des animaux traités par rapport à celui du groupe des animaux de contrôle — est de 4,4 mg/kg par voie orale chez la souris.

Dans le test de stimulus chimique [SIEGMUND E., CADMUS R. GO LU - Proc. Soc. Exp. Biol. Med. 95, 729-731, (1957)] la DE 50 de ce composé — qui représente la dose qui inhibe de 50 % le nombre de crampes abdominales par rapport à celui du groupe des animaux de contrôle — est de 9 mg/kg par voie orale chez la souris.

Associés aux excipients pharmaceutiques habituels, les composés de l'invention peuvent être administrés par voie enthérale ou parenthérale à une dose journalière comprise entre 0,5 et 50 mg par kilo de poids corporel.

Pour l'administration chez l'être humain, on utilisera de préférence une dose journalière comprise entre 100 et 1000 mg selon le mode d'administration.

Selon une réalisation préférée, les composés de l'invention concernent plus particulièrement les composés dans lesquels, seuls ou ensemble les substituants ont les significations suivantes :

$R_1$ représente l'hydrogène ou le radical méthyl,
$R_2$ représente l'hydrogène ou le radical méthyl,
B représente le radical hydroxyméthylène et
A représente un radical aminé secondaire ou tertiaire, et plus particulièrement un radical alkylamino, dialkylamino et benzylamino, ou un radical dans lequel l'atome d'azote de liaison est inclu dans un hétérocycle.

L'invention concerne plus spécialement le composé de formule I dans lequel $R_1$ et $R_2$ représentent l'hydrogène, B représente le radical hydroxyméthylène et A représente un reste isopropylamino.

Exemple 1 : 2-(N-méthyl-N-benzylamino)-1-(3-méthyl-2-benzoxazolinon-6-yl) propanone

A une solution acétonique de 14,2 g (0,05 M) de 2-bromo -1-(3-méthyl-2-benzoxazolinon-6-yl) propanone refroidie à 0°C, on a ajouté 12,1 g (0,10 M) de N-méthylbenzylamine, et laissé la réaction se poursuivre sous agitation en maintenant la température à 0°C pendant 15 heures.
On a ensuite essoré le précipité, concentré le filtrat et filtré le nouveau précipité ; les deux fractions de précipité ont été rassemblées, puis traitées par 250 $cm^3$

d'acide chlorhydrique normal. Après filtration, le filtrat a été alcalinisé par une solution aqueuse de soude à 10 %, le précipité essoré, lavé à l'eau, séché et recristallisé dans de l'éthanol à 95°.
On a ainsi obtenu 13 g de composé du titre ayant pour point de fusion F = 154°C et ayant pour analyse élémentaire :

| | C % | H % | N % |
|---|---|---|---|
| Calculé | 70,35 | 6,21 | 8,63 |
| Trouvé | 70,32 | 6,23 | 8,64 |

Exemple 2 : 2-méthylamino-1-(3-méthyl-2-benzoxazolinon-6-yl) propanone

Dans 100 $cm^3$ de méthanol contenant 0,01 mole d'acide chlorhydrique, on a dissous 3,24 g (0,01 M) du composé obtenu à l'exemple 1, puis ajouté 100 mg de charbon palladié à 10 %, et soumis le mélange à une hydrogénation à température et pression ordinaire jusqu'à absorption du volume théorique d'hydrogène.
La réaction terminée, on a filtré, évaporé le solvant sous vide, et recristallisé dans l'éthanol absolu.
On a obtenu 1,8 g de produit du titre ayant pour point de fusion F = 240°C et pour analyse élémentaire :

| | C % | H % | N % |
|---|---|---|---|
| Calculé | 51,52 | 5,76 | 10,01 |
| Trouvé | 51,52 | 5,49 | 10,05 |

Exemple 3 : 2-isopropylamino 1-(2-benzoxazolinon-6-yl) éthanone

Après avoir dissous 17,7 g (0,3 M) d'isopropylamine dans 45 $cm^3$ de méthanol, on a ajouté 7,7 g (0,03 M) de 2-bromo 1-(2-benzoxazolinon-6-yl) éthanone, et laissé la réaction se poursuivre sous agitation à température ambiante pendant 30 minutes.

On a ensuite essoré le précipité, lavé, séché, puis dissous dans l'éthanol. Le produit du titre a alors été obtenu cristallisé sous forme de chlorhydrate, en faisant passer un courant de gaz chlorhydrique sec. On a ainsi obtenu 4 g de produit ayant pour point de fusion F = 205°C et dont le monohydrate a pour analyse élémentaire :

| | C % | H % | N % |
|---|---|---|---|
| Calculé | 57,14 | 6,34 | 11,11 |
| Trouvé | 57,25 | 6,32 | 11,16 |

Exemple 4 : 2 [4-(2-méthoxyphényl) pipérazinyl] 1-(3-méthyl 2-benzoxazolinon-6-yl) éthanone

Après avoir dissous à chaud 5,4 g (0,02 M) de 2-bromo 1-(3-méthyl-2-benzoxazolinon-1-yl) éthanone dans la quantité suffisante de dioxanne, on a ajouté, goutte à goutte et sous agitation, une solution constituée par 3,56 g (0,02 M) de 2-méthoxy phényl pipérazine et 5 g (0,05 M) de triéthylamine dans 20 $cm^3$ de dioxanne, et on a laissé la réaction se poursuivre à température ambiante pendant 24 heures. On a ensuite essoré le précipité, évaporé le solvant recueilli, joint la deuxième fraction de précipité au premier, traité par 50 $cm^3$ de soude à 10 %, essoré et lavé, puis recristallisé dans l'acétone.
On a alors obtenu 5,3 g de produit du titre ayant pour point de fusion F = 186 - 187°C, et pour analyse élémentaire :

| | C % | H % | N % |
|---|---|---|---|
| Calculé | 66,13 | 6,08 | 11,02 |
| Trouvé | 66,11 | 6,11 | 10,99 |

Exemple 5 : 2-(4-benzyl-pipéridinyl) 1-(3-méthyl 2-benzoxazolinon-6-yl) éthanone

En procédant comme indiqué dans l'exemple 4, mais en remplaçant l'amine indiqué par la 4-benzyl pipéridine, on obtient 5,7 g de produit du titre ayant pour point de fusion F = 168 - 169°C, et pour analyse élémentaire :

| | C % | H % | N % |
|---|---|---|---|
| Calculé | 72,51 | 7,69 | 13,17 |
| Trouvé | 72,56 | 7,65 | 13,17 |

Exemple 6 : 2-amino-1-(2-benzoxazolinon-6-yl) éthanone

Dans un réacteur contenant 1200 $cm^3$ d'éthanol absolu, on a introduit 2,56 g (0,10 M) de 2-bromo 1-(2-benzoxazolinon-6-yl) éthanone et ajouté 14,1 g (0,10 M) d'hexaméthylène tétramine, puis on a porté le mélange à reflux pendant 3 heures.

Une fois la réaction terminée, on a essoré et séché le précipité, puis hydrolysé le mélange par chauffage à reflux en présence de 90 $cm^3$ d'acide chlorhydrique concentré et de 170 $cm^3$ d'éthanol à 95°.

Après refroidissement, on a essoré et séché le précipité, puis recristallisé dans une solution d'acide chlorhydrique (1 partie HCl concentré - 1 partie $H_2O$).

On a ainsi obtenu 6,7 g de composé du titre ayant pour analyse élémentaire :

| | C % | H % | N % |
|---|---|---|---|
| Calculé | 47,28 | 3,96 | 12,25 |
| Trouvé | 47,19 | 3,85 | 12,23 |

Exemple 7 : 2-isopropylamino 1-(2-benzoxazolinon-6-yl) éthanol

On a dissous 2,34 g (0,01 M) du composé préparé conformément à l'exemple 3 dans 100 $cm^3$ de méthanol, puis on a ajouté, sous agitation et par petites fractions, 0,7 g (0,02 M) de borohydrure de sodium, puis laissé sous agitation pendant 4 heures à température ambiante.

On a ensuite évaporé à sec, repris le résidu par de l'alcool absolu et fait barboter un courant d'acide chlorhydrique sec, puis chauffé à ébullition.

Après élimination du chlorure de sodium par filtration, on a laissé recristalliser et recueilli 2 g de composé du titre sous forme de chlorhydrate ayant pour point de fusion F = 220°C et pour analyse élémentaire :

| | C % | H % | N % |
|---|---|---|---|
| Calculé | 52,84 | 6,28 | 10,27 |
| Trouvé | 52,81 | 6,33 | 10,35 |

Exemple 8 : 2- [4-(2-méthoxy phényl) pipérazinyl] 1-(3-méthyl-2-benzoxazolinon-6-yl) éthanol

En suivant le même mode opératoire que celui indiqué à l'exemple 7, mais en partant du composé obtenu à l'exemple 4, on obtient, directement sous forme de base, 3,3 g de produit du titre ayant pour point de fusion F = 126 - 127°C, et pour analyse élémentaire :

| | C % | H % | N % |
|---|---|---|---|
| Calculé | 65,78 | 6,57 | 10,96 |
| Trouvé | 65,54 | 6,58 | 10,95 |

Exemple 9 : 2-(4-benzyl pipéradinyl) 1-(3-méthyl 2-benzoxazolinon-6-yl) éthanol

En procédant comme indiqué dans l'exemple 7, mais en partant du composé obtenu à l'exemple 5, on obtient, directement sous forme de base, 3,2 g de composé du titre ayant pour point de fusion F = 156°C et pour analyse élémentaire :

| | C % | H % | N % |
|---|---|---|---|
| Calculé | 72,10 | 7,15 | 7,64 |
| Trouvé | 71,82 | 7,22 | 7,75 |

Exemple 10 : Thréo-2-[4-(3-trifluorométhyl phényl) pipérazinyl] 1-(3-méthyl-2-benzoxazolinon-6-yl) propanol

Après avoir préparé selon un processus identique à celui décrit dans l'exemple 4 la 2-[4-(3-trifluorométhyl phényl) pipérazinyl] 1-(3-méthyl-2-benzoxazolinon-6-yl) propanone, on a mis 8,67 g de ladite cétone en suspension dans le méthanol, puis on a ajouté sous agitation 1,4 g de borohydrure de sodium par petites fractions. On a ensuite laissé l'agitation se poursuivre pendant une heure à température ambiante. On a essoré le précipité d'amino alcool obtenu, et on l'a recristallisé dans l'alcool absolu pour obtenir 7,4 g de composé du titre ayant pour point de fusion F = 192°C et pour analyse élémentaire :

| | C % | H % | N % |
|---|---|---|---|
| Calculé | 60,68 | 5,56 | 9,65 |
| Trouvé | 60,49 | 5,48 | 9,63 |

Exemple 11 : Erythro 2-[4-(2-méthoxy phényl) pipérazinyl] 1-(3-méthyl-2-benzoxazolinon-6-yl) propanol

Après avoir préparé la 2- [4-(2-méthoxy phényl) pipérazinyl] 1-(3-méthyl-2-benzoxazolinon-6-yl) propanone en suivant le procédé de l'exemple 4, on a mis en suspension 5 g de ladite cétone dans de l'alcool absolu, puis, après avoir ajouté 0,5 g de charbon palladié à 10 %, on a soumis le mélange à une pression d'hydrogène de 80 bars sous agitation, en portant progressivement la température à 100°C. En fin de réaction, on a filtré le catalyseur, évaporé le solvant, recristallisé dans l'alcool absolu et obtenu 3,5 g de produit du titre ayant pour point de fusion F = 183°C et pour analyse élémentaire :

| | C % | H % | N % |
|---|---|---|---|
| Calculé | 66,48 | 6,85 | 10,57 |
| Trouvé | 66,64 | 6,93 | 10,33 |

Exemple 12 : 2-[4-(3-trifluorométhyl) phényl pipérazinyl] 1-(3-méthyl 2-benzoxazolinon-6-yl) éthanol

Dans un premier temps, en suivant le même mode opératoire que celui décrit en détail dans l'exemple 4, on a préparé la 2-[4-(3-trifluorométhyl) phényl pipérazinyl] 1-(3-méthyl 2-benzoxazolinon-6-yl) éthanone. Puis dans une deuxième étape, on a réduit 4 g du composé précédent au moyen du borohydrure de sodium selon le procédé décrit dans l'exemple 7, et obtenu 3,6 g du composé du titre, ayant pour point de fusion F = 139°C et pour analyse élémentaire :

| | C % | H % | N % |
|---|---|---|---|
| Calculé | 59,85 | 5,26 | 9,97 |
| Trouvé | 59,79 | 5,49 | 9,93 |

Selon les mêmes procédés, on a également préparé les composés dont les caractéristiques sont indiquées dans le tableau II ci-après :

TABLEAU II

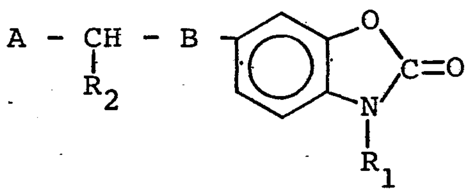

| COMPOSE N° | A | B | $R_1$ | $R_2$ | SEL | F°C |
|---|---|---|---|---|---|---|
| 1 | $-NH-CH_3$ | 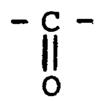 | $-CH_3$ | H | HCl | 255 |
| 2 (Ex. 2) | $-NH-CH_3$ | 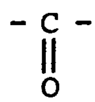 | $-CH_3$ | $-CH_3$ | HCl | 240 |
| 3 | 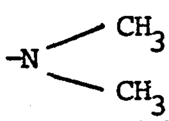 | 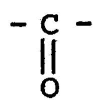 | $-CH_3$ | $-CH_3$ | Base | 90 |
| 4 | 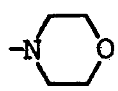 | 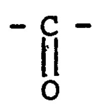 | $-CH_3$ | $-CH_3$ | Base | 168 |
| 5 | 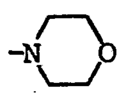 | 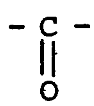 | H | $-CH_3$ | Base | 194 |
| 6 (Ex. 1) | 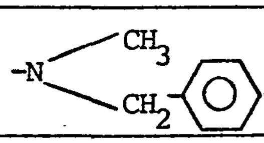 | 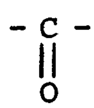 | $-CH_3$ | $-CH_3$ | Base | 154 |
| 7 | 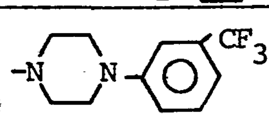 | 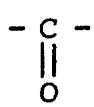 | $-CH_3$ | $-CH_3$ | Base | 115 |
| 8 (Ex. 3) | 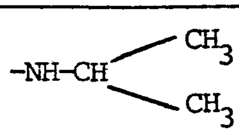 | 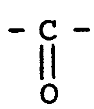 | H | H | HCl | 205 |

TABLEAU II (Suite)

| COMPOSE N° | A | B | $R_1$ | $R_2$ | SEL | F°C |
|---|---|---|---|---|---|---|
| 9 (Ex. 4) | 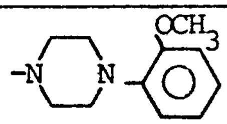 | 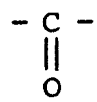 | $-CH_3$ | H | Base | 186 |
| 10 | 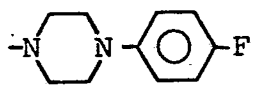 | 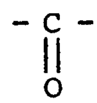 | $-CH_3$ | $-CH_3$ | Base | 193 |
| 11 | 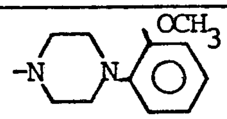 | 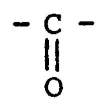 | H | $-CH_3$ | Base | 225 |
| 12 | 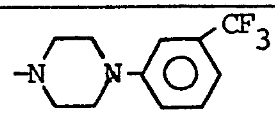 | 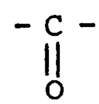 | H | H | Base | 207 |
| 13 (Ex. 5) | 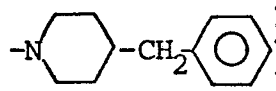 | 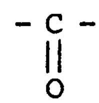 | $-CH_3$ | H | Base | 168 |
| 14 (Ex. 6) | $-NH_2$ | 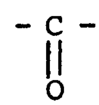 | H | H | HCl | > 260 |
| 15 | 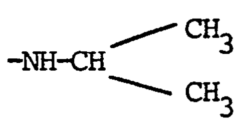 | 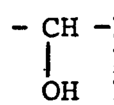 | $-CH_3$ | H | HCl | 237 |
| 16 | $-NH_2$ | 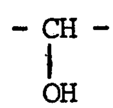 | H | H | HCl | 230 |
| 17 | $-NH-CH_3$ | 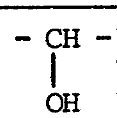 | H | H | HCl | 230 |
| 18 (Ex. 7) | 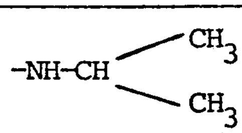 | 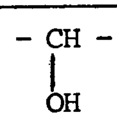 | H | H | HCl | 220 |
| 19 | 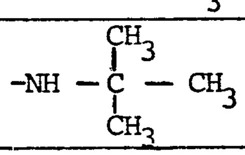 | 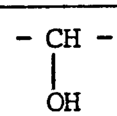 | H | H | HCl | 215 |

TABLEAU II (Suite)

| COMPOSE N° | A | B | $R_1$ | $R_2$ | SEL | F°C |
|---|---|---|---|---|---|---|
| 20 (Ex.10) | 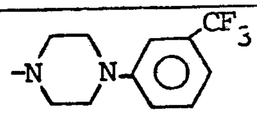 | 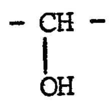 | $-CH_3$ | $-CH_3$ | Base [Thréo] | 192 |
| 21 | 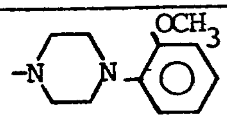 | 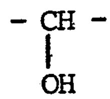 | $-CH_3$ | $-CH_3$ | Base [Thréo] | 232 |
| 22 (Ex. 8) | 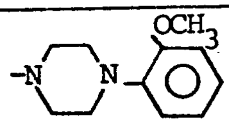 | 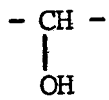 | $-CH_3$ | H | Base | 126 |
| 23 | 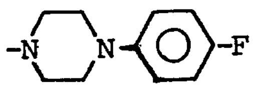 | 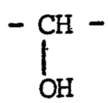 | $-CH_3$ | $-CH_3$ | Base [Thréo] | 217 |
| 25 (Ex. 9) | 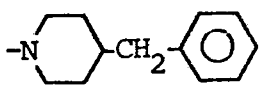 | 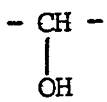 | $-CH_3$ | H | Base | 156 |
| 26 | 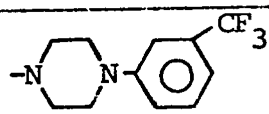 | 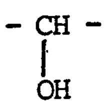 | H | $-CH_3$ | Base [Erythro] | 187 |
| 27 (Ex.11) | 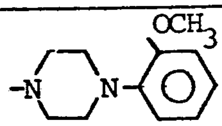 | 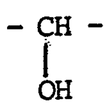 | $-CH_3$ | $-CH_3$ | Base [Erythro] | 183 |
| 28 | 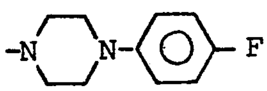 | 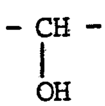 | $-CH_3$ | $-CH_3$ | Base [Erythro] | 157 |
| 29 | 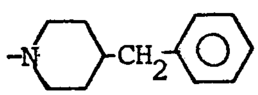 | 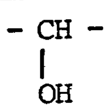 | $-CH_3$ | $-CH_3$ | Base [Erythro] | 181 |
| 30 | 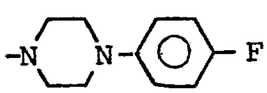 | 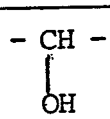 | H | H | Base | 224 |
| 31 | 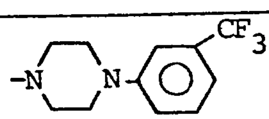 | 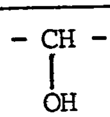 | $-CH_3$ | H | Base | 139 |

0049203

TABLEAU II (Suite)

| COMPOSE N° | A | B | $R_1$ | $R_2$ | SEL | F°C |
|---|---|---|---|---|---|---|
| 32 | 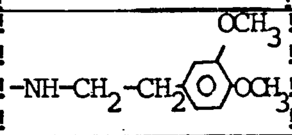 | 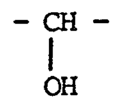 | H | H | HCl | 233 |
| 33 | 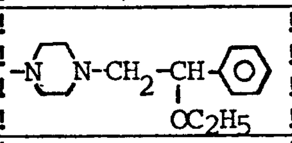 | 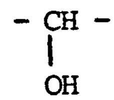 | H | H | 2 HCl | 224 |
| 34 | 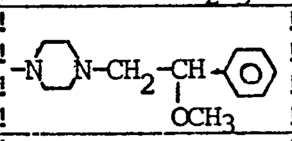 | 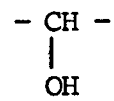 | H | H | 2 HCl | 230 |
| 35 | 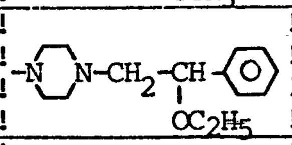 | 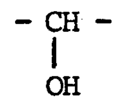 | $-CH_3$ | H | 2 HCl | 230 |
| 36 | 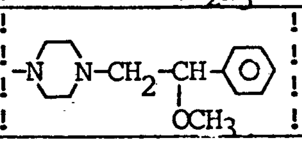 | 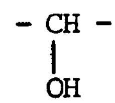 | $-CH_3$ | H | 2 HCl $H_2O$ | 223 |

REVENDICATIONS

1. Composés, caractérisés en ce qu'ils répondent à la formule :

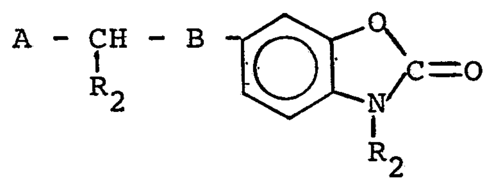

dans laquelle $R_1$ représente l'hydrogène ou un radical alkyle inférieur ; $R_2$ représente l'hydrogène ou un radical alkyle inférieur ; A représente le radical amino ou un radical aminé secondaire ou tertiaire, ou un radical dans lequel l'atome d'azote de liaison est inclus dans un hétérocycle ; et B représente soit le radical carbonyle, soit le radical hydroxyméthylène.

2. Composés selon la revendication 1, caractérisés en ce que A est un radical alkylamino ou benzylamino.

3. Composés selon la revendication 1, caractérisés en ce que A est un radical dans lequel l'atome d'azote de liaison est inclus dans un hétérocycle et est choisi parmi les radicaux pipéridino, morpholino, 4-benzyl pipéridyle, 4-méthyl pipérazinyle, 4-(2-alcoxy-2-phényl-éthyl) pipérazinyl et 4-phényl pipérazinyle dans lequel le groupement aromatique

BAD ORIGINAL

peut être substitué par un halogène, un radical alcoxy inférieur ou le radical trifluorométhyle.

4. Composés selon l'une quelconque des revendications 1 à 3, caractérisés en ce que B représente le radical carbonyle.

5. Composés selon la revendication 4, caractérisés en ce que $R_1$ et $R_2$ représentent le radical méthyle et A représente le radical 4-(4-fluoro) phényl pipérazinyle.

6. Composés selon l'une quelconque des revendications 1 à 3 caractérisés en ce que B représente le radical hydroxyméthylène.

7. Composés selon la revendication 6, caractérisés en ce que $R_1$ et $R_2$ représentent l'hydrogène et A est le radical isopropylamine.

8. Composés selon la revendication 6, caractérisés en ce que $R_2$ représente le radical méthyle et que lesdits composés ont une configuration thréo.

9. Composés selon la revendication 6, caractérisés en ce que $R_2$ représente le radical méthyle et que lesdits composés ont une configuration érythro.

10. Composé selon la revendication 9, caractérisé en ce que A représente le radical 4-(2-méthoxy) phényl pipérazinyle et $R_1$ le radical méthyle.

11. Composés selon la revendication 6, caractérisés en ce que A représente le radical 4-(3-trifluorométhyl) phényl pipérazinyle, $R_1$ est le radical méthyle et $R_2$ est un atome d'hydrogène.

12. Procédé pour l'obtention des composés selon la revendication 1, caractérisé en ce qu'il comprend la condensation d'une amine AH avec une 6-bromacétyl benzoxazolinone substituée par $R_1$ et $R_2$, les radicaux A, $R_1$ et $R_2$ ayant les significations indiquées dans la revendication 1, et si nécessaire la réduction du groupe carbonyle en groupe hydroxyméthylène et éventuellement la transformation en sel pharmaceutiquement acceptable.

13. Procédé selon la revendication 12 pour la préparation d'un composé de formule I dans laquelle A représente un radical aminé secondaire ou tertiaire caractérisé en ce que l'amine

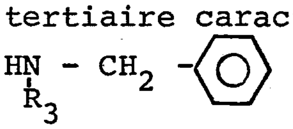

est condensée avec une 6-bromo benzoxazolinone et qu'on élimine ensuite le reste N-benzyl du composé obtenu par hydrogénation catalytique.

14. Procédé selon la revendication 12 pour la préparation d'un composé de formule I dans laquelle A représente le radical - $NH_2$ caractérisé en ce que l'hexaméthylène tétramine est condensée avec une 6-bromo benzoxazolinone puis le produit résultant soumis à une hydrolyse en milieu acide.

15. Composition pharmaceutique, notamment utile pour le traitement de l'hypertension ou des syndromes douloureux, caractérisée en ce qu'elle comprend, associé aux excipients pharmaceutiques habituels, un composé selon l'un quelconque des revendications 1 à 11.

16. Application des composés selon l'une quelconque des revendications 1 à 11 en thérapeutique, notamment dans le traitement de l'hypertension, caractérisée en ce qu'elle comprend l'administration de ces composés, associés aux excipients pharmaceutiques habituels, à des doses journalières comprises entre 0,5 et 50 mg par kilo de poids corporel selon les modes d'administration.

17. Application du composé selon la revendication 12 en thérapeutique notamment dans le traitement des syndromes douloureux caractérisée en ce qu'elle comprend, son administration en association avec des excipients pharmaceutiques habituels, à des doses journalières comprises en 0,5 et 50 mg par kilo de poids corporel selon le mode d'administration.

0049203

Office européen des brevets

# RAPPORT DE RECHERCHE EUROPEENNE

Numéro de la demande
EP 81 40 1497

## DOCUMENTS CONSIDERES COMME PERTINENTS

| Catégorie | Citation du document avec indication, en cas de besoin, des parties pertinentes | Revendication concernée |
|---|---|---|
| X | DE - A - 2 429 253 (BOEHRINGER)<br>* Pages 5,9 (paragraphe 2),17-19, 25-27 et revendications *<br>-- | 1-17 |
| A | FR - A - 2 244 507 (INSERM)<br>* Revendications 1,2,6 *<br>---- | 1,15 |

CLASSEMENT DE LA DEMANDE (Int. Cl.³)

C 07 D 263/58
A 61 K 31/42

DOMAINES TECHNIQUES RECHERCHES (Int. Cl.³)

C 07 D 263/58
A 61 K 31/42

CATEGORIE DES DOCUMENTS CITES

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons

&: membre de la même famille, document correspondant

X Le présent rapport de recherche a été établi pour toutes les revendications

| Lieu de la recherche | Date d'achèvement de la recherche | Examinateur |
|---|---|---|
| La Haye | 03-11-1981 | CHOULY |

OEB Form 1503.1 06.78